(19) **Europäisches Patentamt / European Patent Office / Office européen des brevets**

(11) **EP 4 763 121 A1**

(12) **EUROPEAN PATENT APPLICATION**

(43) Date of publication:
24.06.2026 Bulletin 2026/26

(21) Application number: 24222649.6

(22) Date of filing: 20.12.2024

(51) International Patent Classification (IPC):
A61B 34/20 (2016.01)　　A61B 34/30 (2016.01)
A61B 90/14 (2016.01)　　A61C 1/00 (2006.01)
A61B 17/00 (2006.01)　　A61B 90/00 (2016.01)

(52) Cooperative Patent Classification (CPC):
**A61B 34/20; A61B 34/30; A61B 34/32;
A61B 90/14; A61C 1/082;** A61B 2017/00464;
A61B 2017/00473; A61B 2017/00477;
A61B 2017/00725; A61B 2034/2051;
A61B 2034/2055; A61B 2034/2059; A61B 2034/207;
A61B 2090/3983

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(71) Applicant: **Digicuto**
13008 Marseille (FR)

(72) Inventors:
• KOUBI, Stefan
  13008 Marseille (FR)

• GUREL, Galip
  13008 Marseille (FR)
• TOURBAH, Karim
  13008 Marseille (FR)
• DREVELLE, Xavier
  13008, Marseille (FR)
• VALLET, Arthur
  13008, Marseille (FR)
• MAITREJEAN, Gilles
  13008, Marseille (FR)

(74) Representative: **A.P.I. Conseil**
Technopôle Hélioparc
4, rue Jules Ferry
64000 Pau (FR)

(54) **AUTOMATED MEDICAL PROCEDURE SYSTEM WITH ACCURATE SPATIAL CALIBRATION AND REGISTRATION**

(57) The subject application presents an automated medical procedure system featuring accurate spatial calibration and registration capabilities.

The system comprises a calibrated robotic subsystem with a position-accurate arm, a tracking subsystem, and processor-based modules for registration and calibration.

Key innovations include the use of factory-defined geometric configurations for targets, systematic spatial recalibration before each procedure, and the ability to perform both rigid point-to-point and point cloud registrations.

These features enable the system to maintain accurate spatial relationships between components and patient anatomy, allowing for highly accurate and adaptable automated medical procedures across various specialties.

[Fig. 1]

Fig. 1

EP 4 763 121 A1

## Description

## Technical Field

The subject application relates to the field of medical robotics, specifically to automated systems for performing medical procedures. This subject application addresses the need for highly accurate and adaptable robotic systems in various medical specialties, including but not limited to dentistry, orthopedics, and minimally invasive surgeries.

## Background Art

Existing medical robotic systems often require significant human intervention and lack the ability to adapt to dynamic surgical environments. These systems typically rely on pre-operative imaging and manual calibration, which can lead to inaccuracies during procedures.

Current systems face challenges in maintaining accurate spatial relationships between robotic components and patient anatomy throughout medical procedures. This is particularly problematic in scenarios where patient movement or tissue deformation occurs.

Moreover, traditional systems often struggle with real-time recalibration and registration, limiting their ability to perform complex procedures automatedly and with high accuracy.

These limitations can result in less accurate surgical interventions, potentially leading to suboptimal patient outcomes and increased risk of complications.

Furthermore, the reliance on manual calibration and the inability to adapt in real-time can extend procedure durations, increasing patient discomfort and surgical team fatigue.

The lack of automated adaptation capabilities may also restrict the range of procedures that can be performed robotically, limiting the potential benefits of robotic assistance in complex surgical scenarios.

These drawbacks underscore the need for a more advanced and efficient solution to the technical problem of maintaining accurate spatial relationships and adaptability in automated medical robotic systems within a timeframe acceptable for the targeted clinical workflow. While other systems may achieve similar levels of performance, their calibration times are often too long to be practically integrated into the intended clinical procedures.

## Summary of the subject application

As described in the accompanying claims, the subject application provides methods, systems and apparatuses for performing automated medical procedures, establishing precise and accurate spatial relationships between components, and maintaining accurate calibration and registration for enhanced medical interventions.

Dependent claims describe specific embodiments of the subject application.

These and other aspects of the subject application will be apparent from an elucidated based on the embodiments described hereinafter.

## Brief Description of Drawings

Further details, aspects and embodiments of the subject application will be described, by way of example only, with reference to the drawings. In the drawings, like reference numbers are used to identify like or functionally similar elements. Elements in the figures are illustrated for simplicity and clarity and have not necessarily been drawn to scale. Elements indicated by a solid line are mandatory, while elements indicated by a dotted line are optional.

Figure 1 shows a block diagram illustrating an automated medical system according to the subject application.

Figure 2 shows a rigid frame illustrated as a head holder according to the subject application.

Figure 3 shows a detachable element according to the subject application.

Figure 4 shows dedicated probe according to the subject application.

Figure 5 shows a calibration process of the detachable element of figure 4 with the dedicated probe of figure 5.

Figure 6 shows an illustration of a rigid point-to-point registration according to the subject application.

Figure 7 shows an illustration of a rigid point cloud registration according to the subject application.

Figure 8 shows a schematic flow diagram of a computer-implemented method according to the subject application.

## Description of Embodiments

### ▶Preliminary Remarks

Because the illustrated embodiments of the subject application may, for the most part, be composed of components known to the skilled person, details will not be explained in any greater extent than that considered necessary for the understanding and appreciation of the underlying concepts of the subject application, in order not to obfuscate or distract from the teachings of the subject application.

### ▶Structure of the Description

The description of the subject application is organized in a logical and comprehensive manner to facilitate understanding of the automated medical procedure system.

It begins with an overview of the system's purpose and key components.

The description then delves into detailed explanations of each major subsystem, including the calibrated robotic subsystem, the tracking subsystem, and the processor-based modules for registration and calibration.

Each component is thoroughly defined, with specific attention given to technical terms and their contextual meanings within the subject application.

The description progresses to outline the system's operational methods, including calibration, registration, and control processes.

Throughout the text, examples are provided to illustrate the application of various components and processes in different medical scenarios, enhancing the reader's comprehension of the system's versatility and precision in automated medical procedures.

▶**Objective of the subject application**

One of the primary objectives of the subject application is to provide an automated medical procedure system capable of maintaining accurate spatial relationships between its components and the patient's anatomy throughout the entire procedure.

To achieve this, the inventors propose an innovative system that integrates a calibrated robotic subsystem, a tracking subsystem, and sophisticated processor-based modules for registration and calibration.

This solution aims to enable the system to perform automated medical procedures with minimal human intervention, while continuously adapting to changes in the surgical environment.

The subject application specifically addresses the need for real-time recalibration and registration, allowing for enhanced precision and accuracy in various medical procedures, from dental surgeries to complex orthopedic interventions.

▶**Automated Medical procedure System with Accurate Spatial Component Relationships**

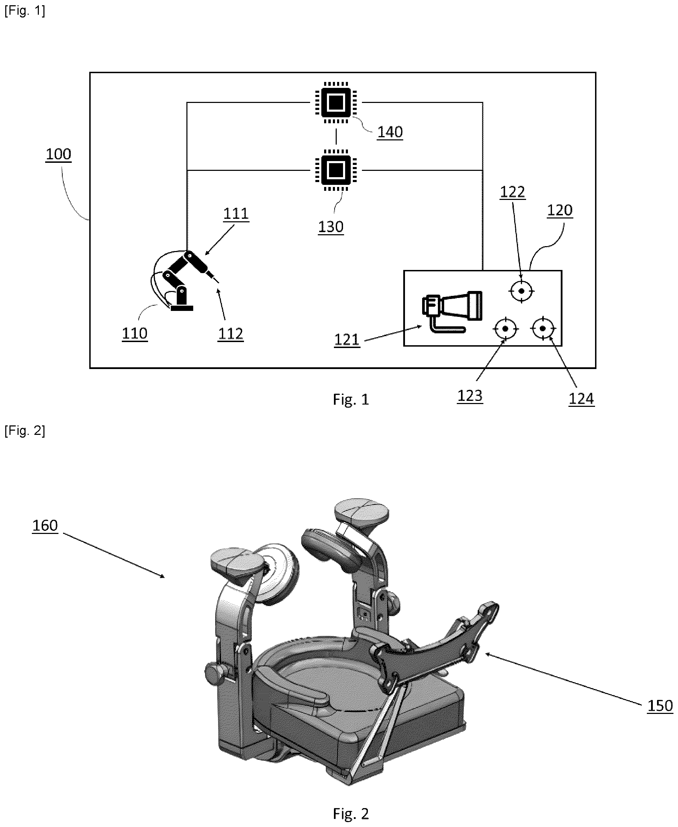

As illustrated in figure 1, the subject application relates to a system 100 for performing automated medical procedures, and establishing accurate spatial relationships between components.

The term "automated" refers to the capability of the system 100 to perform medical procedures with minimal human intervention, following pre-programmed instructions and utilizing real-time data from various sensors

For example, the term "automated" may refer to a robotic arm's ability to execute a predefined toolpath without direct human guidance, the system 100's capacity to perform spatial recalibration according to programmed parameters before each medical procedure, or the ability to track and compensate for minor patient movements during the medical procedure within predetermined limits.

The term "medical procedure" encompasses a wide range of diagnostic, therapeutic, or surgical interventions performed on a patient using the automated system 100. These medical procedures are specifically designed to be carried out with high precision and repeatability by the system 100. For instance, the system 100 is capable of performing these procedures with a precision under 200 to 500 microns, preferably around 300 microns, when measured using a tracking system, ensuring a level of accuracy that is difficult to achieve consistently with manual techniques.

For example, the term "medical procedures" may include dental surgeries such as implant placement, crown preparation or veneer preparation, orthopedic medical procedures like joint replacements, or minimally invasive surgeries in various medical specialties.

The term "accurate" refers to the quality of a measurement or positioning that closely matches the true or intended target value. In the context of spatial relationships between medical system components, accuracy characterizes the ability to consistently reach or identify the correct target position.

For example, in a targeting task, an accurate system would reliably position instruments at the exact intended anatomical location, similar to hitting the bullseye in target practice. This differs from precision, which only indicates repeatability without necessarily achieving the correct target position. An accurate medical system ensures that when a specific anatomical target is designated, the system's components will be positioned at the genuine intended location rather than just repeatedly reaching the same incorrect position.

In the subject application, the system 100 comprises several components: a calibrated robotic subsystem 110, a tracking subsystem 120, a processor-based registration module 130, and a processor-based calibration module 140.

▶▶**the calibrated robotic subsystem**

In practice, the calibrated robotic subsystem 110 includes a position-accurate robotic arm 111.

The term "calibrated" in "calibrated robotic subsystem 110" refers to the process of establishing and maintaining accurate spatial relationships between the position-accurate robotic arm 111, its tools, and the tracking subsystem 120. This calibration ensures that the movements of the position-accurate robotic arm 111 correspond accurately to the intended actions in the patient's anatomy.

For example, the term "calibrated" may refer to the initial factory setup that defines the baseline spatial relationships between system 100 components, the ongoing process of comparing current geometries to factory-defined configurations, or the act of updating the system 100's spatial model based on recalibration results before each medical procedure.

In particular, the position-accurate robotic arm 111 refers to an articulated mechanical arm that is capable of executing accurate movements and manipulations in three-dimensional space. This position-accurate robotic arm 111 is designed to handle various medical tools 112 with a high degree of accuracy and repeatability.

For example, the position-accurate robotic arm 111 may refer to a multi-jointed robotic appendage capable of positioning a dental drill with sub-millimeter accuracy, a position-accurate robotic arm 111 designed to guide a surgical scalpel along a complex three-dimensional path, or a robotic manipulator that can delicately handle and position various surgical instruments during a medical procedure.

These position-accurate robotic arms 111 typically feature six degrees of freedom, allowing for comprehensive movement and positioning in three-dimensional space, which enhances their versatility and accuracy in performing complex medical procedures.

Specifically, the position-accurate robotic arm 111 is designed to operate with interchangeable medical tools 112, each tool 112 having a defined tool center point, TCP.

The term "medical tool" refers to any instrument or device that can be attached to and operated by the position-accurate robotic arm 111 to perform specific medical tasks. These tools are designed to be interchangeable, allowing the system 100 to perform various medical procedures.

For example, the term "medical tool" may include a high-speed dental drill for preparing teeth for crowns or veneers, a specialized probe for taking accurate measurements of anatomical structures, or a surgical cutting instrument for performing incisions or resections.

The term "tool center point (TCP)" refers to both a specific point on the medical tool 112 and its associated orientation that are used as references for positioning and movement during medical procedures. While in conventional robotics TCP typically refers only to a position point, in the context of this application, TCP encompasses both the position of a reference point and the orientation of the tool 112 relative to a reference coordinate system. This point is typically located at the functional end of the tool 112 and serves as the focus for the position-accurate robotic arm 111's movements and the system 100's spatial calculations.

For example, the term "tool center point (TCP)" may refer to the tip of a dental drill bit, the end of a surgical probe, or the cutting edge of a scalpel blade attached to the position-accurate robotic arm 111.

Furthermore, the position-accurate robotic arm 111 is capable of automatedly guiding at least one mounted tool 112 along a predefined toolpath linked to an anatomical structure of interest of a patient for executing medical procedures, utilizing the tool's TCP as a reference point.

The term "toolpath" refers to a predefined trajectory or set of movements that the medical tool 112, guided by the position-accurate robotic arm 111, follows during a medical procedure. This path is typically planned based on pre-operative imaging and the specific requirements of the medical procedure.

For example, the term "toolpath" may refer to the accurate route a dental drill follows to prepare a tooth for a crown or a veneer, the sequence of movements a surgical instrument makes to perform a complex dissection, or the path a measurement probe takes to gather data about an anatomical structure of interest.

The term "anatomical structure of interest" refers to the specific part of the patient's body that is the focus of the medical procedure. This structure is typically identified and modeled in the system 100's three-dimensional digital representation of the patient.

For example, the term "anatomical structure of interest" may refer to a specific tooth or group of teeth in a dental medical procedure, a joint such as a knee or hip in an orthopedic surgery, or a particular organ or tissue targeted in a minimally invasive medical procedure.

### ►►the tracking subsystem

In the subject application, the tracking subsystem 120 consists of a tracking device 121 and at least two targets. The tracking can be implemented through various technologies, such as optical tracking, electromagnetic tracking, or other position sensing technologies.

### ►►►the tracking device

In practice, the tracking device 121 is a specialized instrument that is designed to detect, monitor, and record the position and movement of multiple targets in real-time. This device utilizes advanced tracking technology to accurately locate and track the spatial coordinates of specific markers 10 or patterns attached to various components of the system 100.

For example, the tracking device 121 may refer to a high-resolution camera system that uses infrared light to detect reflective markers, a stereoscopic vision system that creates a 3D map of the operating field, an electromagnetic tracking system using field generators and sensors, a laser-based tracking system that measures the exact position of targets, or any other position sensing technology capable of providing accurate spatial tracking.

Furthermore, the term "target" refers to a specifically designed object or set of markers 10 that can be detected and tracked by the tracking device 121. These targets have a predefined geometric configuration that allows the tracking subsystem 120 to determine their accurate position and orientation in three-dimensional space.

For example, the term "target" may refer to a cluster of reflective spheres arranged in a unique pattern, electromagnetic sensors with specific configurations, a flat marker with a distinctive pattern, or any other trackable element compatible with the chosen tracking technology .

In particular, the tracking device 121 is designed to track multiple targets simultaneously.

In other words, the tracking corresponds to a continuous process of detecting, identifying, and monitoring the position and movement of multiple targets simultaneously within the system 100's workspace. This tracking involves real-time data acquisition and processing to maintain an accurate understanding of the spatial relationships between various components of the system 100, regardless of the specific tracking technology employed.

For example, the tracking may refer to continuously updating the coordinates of multiple surgical instruments during a medical procedure, monitoring patient movement to adjust the position-accurate robotic arm 111's position accordingly, simultaneous tracking of both the position-accurate robotic arm 111 and the patient's anatomy to maintain accurate alignment, or using any suitable tracking technology that achieves the required accuracy and real-time performance.

Additionally, the tracking device 121 is designed to be positioned and fixed at a distance of at least a predetermined value from the patient, wherein the predetermined value is selected to allow both tracking and sufficient workspace for the position-accurate robotic arm 111. This predetermined value may vary depending on the specific tracking technology used.

The term "positioned and fixed" refers to the specific placement and securing of the tracking device 121 within the system 100's setup. This positioning is carefully determined to ensure optimal tracking performance of all relevant targets while maintaining a safe and unobstructed workspace for the position-accurate robotic arm 111 and medical personnel.

For example, the term "positioned and fixed" may refer to mounting the tracking device 121 on a stable overhead boom above the operating table, securing the tracking device 121 to a dedicated stand at a specific angle and distance from the patient, such as at a distance greater than 100 cm, preferably 130 cm or 150 cm, integrating the tracking device 121 into the ceiling of a specially designed operating room to provide comprehensive coverage of the surgical field, positioning electromagnetic field generators at optimal locations, or any other configuration that ensures reliable tracking performance while maintaining the required workspace.

In the subject application, the targets are designed to be tracked without physical connection to the calibrated robotic subsystem 110.

The term "tracked without physical connection" refers to the ability of the tracking subsystem 120 to monitor and record the position and movement of targets without requiring any direct mechanical or electrical connection between the targets and the calibrated robotic subsystem 110. While some tracking technologies may require power or signal cables, the tracking itself operates without mechanical linkage to the robotic system, enhancing flexibility and adaptability.

For example, the term "tracked without physical connection" may refer to optical targets detected by cameras without physical linkage, electromagnetic sensors that communicate wirelessly with their field generator, markers that can be freely interchanged and tracked without mechanical coupling to the robotic system, or any other tracking method that maintains spatial tracking without mechanical connection to the robotic subsystem.

Notably, each target has a factory-defined geometric configuration created by an arrangement of multiple tracking markers 10, which typically consists of a specific number of markers 10, such as three or more, arranged in a predefined pattern to enable accurate spatial tracking. The specific arrangement and number of markers depends on the tracking technology employed, but typically consists of a configuration that enables accurate spatial tracking and unique identification of each target.

The term "factory-defined" refers to a predetermined and standardized configuration or characteristic of a component, which is established during the manufacturing process and serves as a reference point for the system 100's calibration and operation. This factory-defined attribute ensures consistency and reliability across different units of the same component and provides a baseline for the system 100 to perform accurate measurements and adjustments, regardless of the tracking technology used.

As an example, the term "factory-defined" may designate the specific arrangement and spacing of markers on a target, the electromagnetic properties and configuration of tracking sensors, the exact dimensions and geometry of a rigid body marker cluster, the calibration parameters of various tracking elements, or any other standardized configuration that enables reliable tracking and system calibration.

The use of factory-defined configurations is essential for maintaining the high precision required in automated medical procedures. These predefined parameters allow the system 100 to detect any deviations from the original specifications, which may occur due to wear, environmental factors, or accidental alterations. By comparing the current state of components to their factory-defined characteristics, the system 100 can perform accurate recalibrations and maintain the spatial relationships necessary for safe and effective operation.

Furthermore, the term "tracking marker" refers to a specific element or feature of a target that is designed to be easily and accurately detected by the tracking device 121. These markers 10 are specifically designed for the chosen tracking technology to ensure reliable tracking.

For example, the term "tracking marker" may refer to a spherical retroreflective ball for optical tracking, an electromagnetic sensor coil, a flat disc with a high-contrast geometric pattern, an active signal emitter, or any other element that enables precise position and orientation tracking with the selected technology.

The configuration of each target allows the tracking device 121 to locate each target within a three-dimensional digital model of the workspace associated with the anatomical structure of interest, built from data acquired by the tracking device 121.

The term "three-dimensional digital model" refers to a virtual environment that accurately reflects the spatial relationships between the patient's anatomy, the position-accurate robotic arm 111, and other elements of the system 100. This model is continuously updated based on real-time tracking data, regardless of the tracking technology employed.

For example, the term "three-dimensional digital model" may refer to a detailed volumetric rendering of a patient's skull and brain based on preoperative CT or MRI scans, updated in real-time with intraoperative tracking data, a dynamic model of a patient's knee joint that incorporates both preoperative imaging and live tracking information for accurate robotic-assisted surgery, a comprehensive spatial map of the operating room that includes the positions of all tracked instruments, the patient, and the position-accurate robotic arm 111 for enhanced situational awareness and procedure planning, or any other digital representation that maintains accurate spatial relationships using tracking data.

### ▶▶▶the targets

In the subject application, the targets include at least one robot target 122 and at least one patient target 123.

Specifically, the robot target 122 has a first factory-defined geometric configuration and is designed to be attached to the position-accurate robotic arm 111. The specific implementation of this target depends on the tracking technology selected but must maintain the required accuracy for robotic positioning.

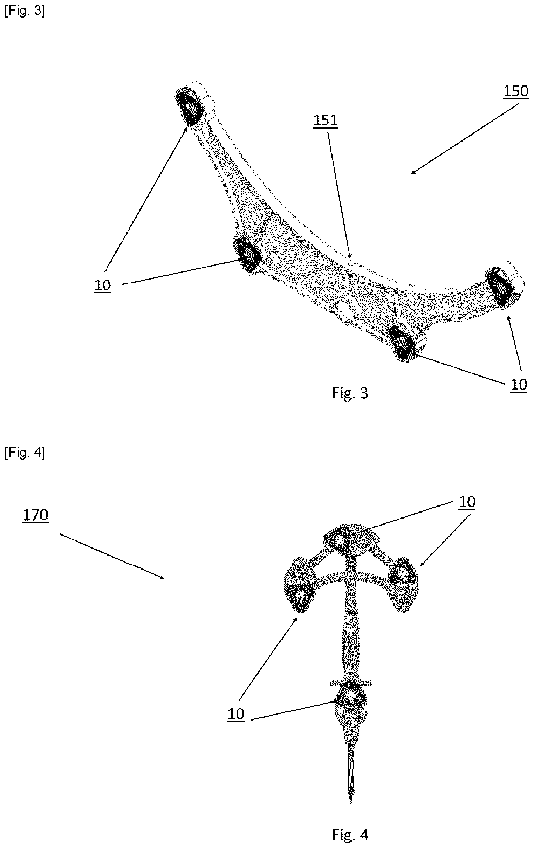

On the other hand, the patient target 123 has a second factory-defined geometric configuration different from the first factory-defined geometric configuration and is designed to be attached to or in proximity to a detachable element 150 fixedly secured to the anatomical structure of interest, as illustrated in figure 2 and figure 3. The patient target's configuration is optimized for the chosen tracking technology while ensuring patient comfort and stability.

The term "attached to or in proximity to" describes two possible configurations for positioning the patient target relative to the anatomical structure of interest, applicable across different tracking technologies. The first configuration, "attached to," indicates a direct physical connection between the patient target and the detachable element. The second configuration, "in proximity to," allows for a close spatial association without necessitating direct contact, providing flexibility in target placement while maintaining accurate tracking.

As an example, the term "attached to or in proximity to" may designate a patient target directly mounted on a dental splint for oral procedures, electromagnetic sensors integrated into a headband, tracking markers positioned on a nearby reference frame, or any other configuration that maintains a stable spatial relationship with the patient's anatomy .

The detachable element 150 is an intermediary between the patient's body and the patient target, that provides a stable reference point for the tracking subsystem 120. The detachable nature of this element allows for its easy removal after the procedure and enables its potential reuse or replacement, while being compatible with the chosen tracking technology.

As an example, the detachable element 150 may include a custom-molded dental tray with integrated tracking markers or sensors and that fits over a patient's teeth during maxillofacial procedures, a non-invasive skull clamp with mounting points for various types of targets and used in neurosurgical interventions, a specially designed bracket compatible with different tracking technologies and temporarily attached to a bone surface during orthopedic surgeries, or any other removable element that provides stable target mounting while ensuring accurate tracking.

### ▶▶the processor-based registration module

In the subject application, the processor-based registration module 130 is designed to perform a registration process that systematically aligns coordinate systems and establishes transformations between the position-accurate robotic arm 111, the tracking device 121, and the anatomical structure of interest.

The term "registration process" refers to a fundamental computational procedure in the system 100 which establishes accurate spatial relationships between different components and reference frames. This process ensures that all elements of the system 100 operate within an unified spatial framework, which is essential for the accurate execution of medical procedures.

As an example, the term "registration process" may designate the act of aligning a pre-operative CT scan of a patient's spine with the real-time position of the patient on the operating table, using markers 10 and anatomical landmarks. It may also include the procedure of mapping the workspace of the robotic arm to the coordinate system 100 of the tracking device, ensuring that the movements of the arm correspond accurately to the tracked positions in three-dimensional space. Additionally, the term "registration process" may comprise the calibration routine that establishes the relationship between the tip of a surgical instrument and the markers 10 attached to its body, allowing for accurate tracking of the instrument's position during a procedure.

▶▶the processor-based calibration module

In the subject application, the processor-based calibration module 140 includes at least a memory and is designed to perform a recalibration process.

As part of this process, the processor-based calibration module 140 stores, in the memory, an initial factory calibration establishing a spatial relationship between the calibrated robotic subsystem 110 and the tracking subsystem 120.

Moreover, the processor-based calibration module 140 performs systematic spatial recalibration of the calibrated robotic subsystem 110 and the tracking subsystem 120 as a preparatory step prior to initiating each medical procedure, as well as upon any tool 112 exchange.

During this recalibration process, the processor-based calibration module 140 compares the current physical geometry of each target to its factory-defined geometric configuration to establish a new spatial relationship.

Subsequently, the module generates new recalibration results based on the comparison and implements the new spatial relationship between the calibrated robotic subsystem 110 and the tracking subsystem 120 using these new recalibration results.

Finally, the processor-based calibration module 140 proceeds with the medical procedure using the newly spatially recalibrated system 100.

▶Rigid frame for Anatomical Structure Immobilization in Automated Medical procedures

In an embodiment of the subject application, as illustrated in figure 2, the system 100 further comprises a rigid frame 160.

The term "rigid frame" refers to a sturdy and inflexible structure that is designed to securely hold the anatomical structure of interest in a fixed position during medical procedures. The rigid frame 160 works in conjunction with the tracking subsystem 120 and the position-accurate robotic arm 111 to maintain a stable reference point for the medical procedure.

For example, the term "rigid frame" may refer to a head frame used in neurosurgical medical procedures to keep the patient's skull perfectly still, a dental bite block that secures the patient's jaw in a specific position for oral surgeries, or a specialized body restraint system 100 used in orthopedic medical procedures to immobilize a limb or joint.

Specifically, this rigid frame 160 is designed to immobilize the anatomical structure of interest.

The term "immobilize" refers to the act of preventing or significantly restricting movement of the anatomical structure of interest during the medical procedure. This immobilization ensures that the pre-operative planning and intra-operative guidance remain accurate throughout the medical procedure.

For example, the term "immobilize" may encompass the action of securing a patient's spine in a fixed position for a delicate spinal surgery, restraining a patient's chest to minimize respiratory movement during a cardiothoracic medical procedure, or fixing a patient's eye in place for an ophthalmic intervention.

The goal of immobilization is to create a stable and predictable environment for the automated system 100 to operate with maximum precision and safety.

Furthermore, the purpose of this immobilization is to minimize unintended patient movement during medical procedures.

▶Dual-Configuration Probe System with Integrated Tracking for Automated Medical procedures

In an embodiment of the subject application, the system 100 further comprises at least one probe.

The term "probe" refers to a specialized instrument within the automated system 100 that is designed for accurate measurement, data collection, or interaction with the anatomical structure of interest.

For example, the term "probe" may refer to a high-precision dental measurement tool 112 that can be attached to the position-accurate robotic arm 111 for automated scanning of a patient's dentition. It may also encompass a handheld surgical probe with markers 10 that can be tracked by the system 100 for manual exploration or measurement during a medical procedure. Additionally, the term "probe" could include a specialized tissue sampling instrument that can be used both automatedly by the position-accurate robotic arm 111 and manually by a surgeon, with the system 100 tracking its position in either case.

Furthermore, in the context of orthopedic medical procedures, the "probe" might refer to a device used for accurate bone surface mapping, capable of being operated by the position-accurate robotic arm 111 or used as a handheld tool 112 for more intuitive surgeon-guided measurements.

Specifically, this probe exists in two distinct configurations, which enhances the system 100's versatility in various medical procedures.

In its first configuration, the probe functions as one of the interchangeable medical tools 112 that can be attached to the position-accurate robotic arm 111. This configuration features a defined tip that serves as a reference point for the system 100's spatial calculations and movements.

Alternatively, in its second configuration, as illustrated in figure 4, the probe operates as a dedicated instrument 170 that is associated with a third target, referred to as the pointer target 124. This dedicated probe 170 is integrated into the tracking subsystem 120 and also possesses a defined point at its tip.

The dual nature of the probe's configuration provides the system 100 with enhanced flexibility in its probing capabilities, allowing for both automated and manual data collection or interaction with the patient's anatomy.

## ▶Calibrated Recess System for Accurate Tip Position Determination in Automated Medical procedures

In an embodiment of the subject application, as illustrated in figure 3, the detachable element 150 comprises at least one calibrated recess 151 with predetermined characteristics, which may include a predetermined depth, shape, position, orientation, and material properties.

The term "recess" refers to an accurately engineered cavity or indentation within the detachable element 150. This recess 151 is specifically calibrated with predetermined characteristics to interact with the probe tip in a controlled manner.

For example, the term "recess" may refer to an accurately machined conical depression in a dental implant guide that allows a probe to rotate and establish its exact tip position relative to the patient's jaw. It may also encompass a spherical indentation on a bone-mounted reference marker 10 used in orthopedic surgeries, which enables the system 100 to calibrate different probe types by analyzing their rotational behavior within the recess 151.

Additionally, the term "recess" could include a multi-faceted cavity in a neurosurgical reference frame that accommodates various probe tip geometries, allowing the system 100 to determine the precise tip position for both robotic-arm-mounted tools and handheld probes.

Furthermore, in the context of minimally invasive medical procedures, the "recess" might refer to a specialized port in a surgical template that allows for the calibration and position tip determination of endoscopic probes and instruments.

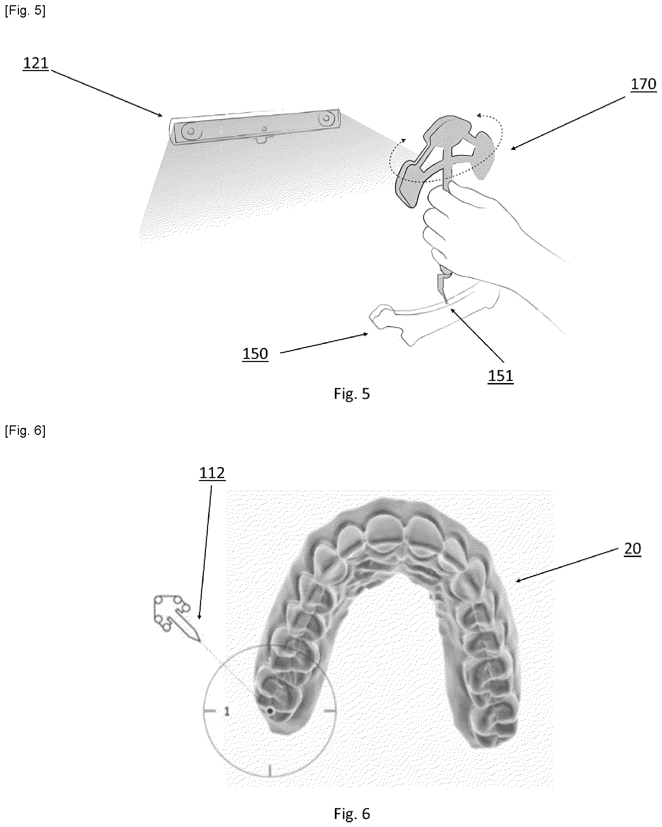

Specifically, as illustrated in figure 5, the calibrated recess 151 is structured to engage all or part of the probe tip and allow rotational movement of the probe when the probe tip is engaged in the recess 151. As a result of this design, the tracking device 121 can determine the exact position of the probe tip in response to the rotation.

In other words, the recess 151 acts as a reference point for the system 100, providing a consistent and known environment for probe interaction and measurement.

Furthermore, the tracking device 121 determines the probe tip position from the detected rotational characteristics in conjunction with either the robot target 122 or the pointer target 124. The choice between these targets depends on whether the probe is one of the interchangeable medical tools 112 or a dedicated probe 170.

This dual functionality enables accurate position tip determination for different probe configurations.

The process of determining the accurate probe tip position using the calibrated recess system can be further elucidated by examining the principles of pivot calibration. This method, which is fundamental to the system 100's ability to accurately locate the tip of various probes, leverages the geometric properties of rotational movement to establish the spatial relationship between the tracked markers 10 and the probe tip.

In the context of the system 100, the calibrated recess 151 serves as a pivoting point for the probe. When the probe tip is engaged in the recess 151, it creates a fixed point around which the rest of the probe rotates.

The tracking device 121 captures the movement of the markers 10 on the probe as it rotates within the recess 151. Each position of the markers 10 during this rotation corresponds to a point on the surface of an imaginary sphere. The center of this sphere represents the probe tip position, which remains stationary at the bottom of the recess during the rotational movement.

To determine the probe tip position, the system 100 employs a least-squares fitting algorithm to solve the equation of the sphere formed by the marker 10 positions. The general form of this equation is:

$$(x - x0)^2 + (y - y0)^2 + (z - z0)^2 = r^2$$

where (x0, y0, z0) represents the coordinates of the sphere's center (i.e., the probe tip position), and r is the radius of the sphere (i.e., the distance from the markers 10 to the probe tip).

The system 100 collects multiple data points as the probe rotates, forming a system of equations that can be solved to find the optimal values for x0, y0, and z0. These coordinates directly correspond to the position of the position of the tip in the tracking device's coordinate system.

The precision of this method is enhanced by the carefully designed characteristics of the calibrated recess 151. The predetermined depth, shape, and orientation of the recess ensure that the probe tip is consistently positioned at the same point, reducing variability in the calibration process. The material properties of the recess may also contribute to the stability of the pivot point, potentially mitigating issues related to tip sharpness or surface deformation that can affect traditional pivot calibration on flat surfaces.

The system 100's ability to use either the robot target 122 or the pointer target 124 for probe tip position determination adds flexibility to the calibration process. When using the robot target 122, the system 100 can directly relate the probe tip position to the robotic arm's coordinate system, facilitating accurate control of tool movements. Conversely, when using the pointer target 124, the system 100 can calibrate handheld probes or verify the calibration of robotic tools independently of the arm's position.

The accuracy of the probe tip position determination is further improved by the system 100's ability to collect and process a large number of data points during the rotational movement. This high volume of data allows the system to employ robust known statistical methods to identify and exclude potential outliers, thereby enhancing the reliability of the calibration.

While traditional pivot calibration methods may be susceptible to errors due to tip shape or surface properties, the use of the calibrated recess 151 mitigates these issues. The accurately engineered characteristics of the calibrated recess 151 ensure a consistent pivot point, reducing the impact of variations in tool tip geometry or wear over time. This consistency is particularly valuable in maintaining calibration accuracy across multiple procedures or extended surgical sessions.

It is important to note that the system 100's calibration process extends beyond mere probe tip position determination. The comprehensive spatial recalibration performed by the processor-based calibration module 140 integrates the probe tip position information with the broader spatial relationships between the robotic subsystem 110 and the tracking subsystem 120. This integration ensures that the probe tip is accurately positioned within the overall surgical workspace, enabling accurate navigation and tool manipulation during medical procedures.

In conclusion, the calibrated recess system for probe tip position determination represents a significant advancement in the field of systems. By combining the principles of pivot calibration with accurately engineered physical references and sophisticated software algorithms, the system achieves a level of accuracy and reliability that is essential for performing complex medical procedures with minimal human intervention.

►**Rigid Point-to-Point Registration System for Accurate Spatial Alignment of Physical Anatomy with 3D Digital Models in Automated Medical procedures**

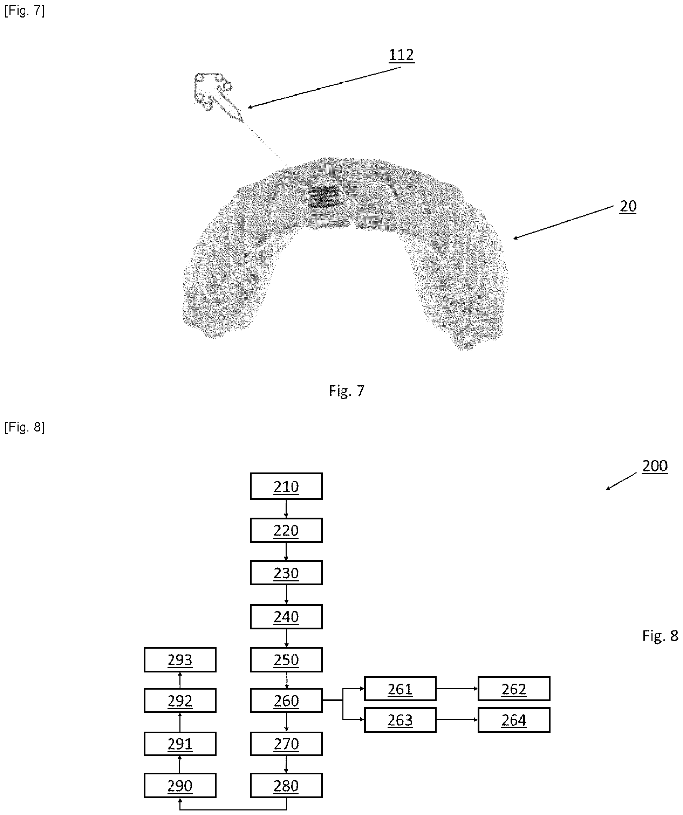

In an embodiment of the subject application, as illustrated in figure 7, the processor-based registration module 130 is further designed to perform a specific function in response to touching predefined registration points on the anatomical structure of interest with the probe tip, where the number of predefined registration points may be, for instance more than three.

Specifically, the module performs a rigid point-to-point registration between the acquired registration points and corresponding predefined points on a three-dimensional digital model 20 of the anatomical structure of interest.

The term "rigid point-to-point registration" refers to an accurate computational process used by the automated system 100 to establish an exact spatial correspondence between specific points on the physical anatomical structure of the patient and their corresponding points in the digital model.

For example, the term "rigid point-to-point registration" may refer to the process of touching specific landmarks on a patient's skull with the probe and aligning these points with corresponding markers 10 in a CT scan for neurosurgical planning. It may also encompass the act of registering key points on a patient's knee joint to a pre-operative MRI model for robotic-assisted orthopedic surgery.

Additionally, this term could include the registration of dental implant sites on a patient's jaw to a 3D model derived from cone beam CT imaging for accurate implant placement.

Furthermore, in the context of spinal surgery, "rigid point-to-point registration" might involve matching vertebral landmarks touched by the probe to their corresponding points on a spinal column model for accurate screw placement guidance.

The term "three-dimensional digital model of the anatomical structure of interest" refers to a detailed, computer-generated representation of the specific part of the patient's body that is the focus of the medical procedure, as illustrated in figure 6 and figure 7.

This digital model 20 is typically created from pre-operative imaging data such as CT, MRI or optical scans and serves as a virtual reference for the automated system 100.

The model contains accurate spatial information about the anatomy, including the location of important structures, landmarks, and the predefined points used in the registration process.

This digital representation allows the system 100 to plan and simulate medical procedures, guide the position-accurate robotic arm 111, and provide real-time feedback during the actual medical intervention.

For example, the term "three-dimensional digital model of the anatomical structure of interest" may refer to a highly detailed virtual representation of a patient's heart, including its chambers, valves, and coronary arteries, used for planning and guiding minimally invasive cardiac medical procedures. It may also encompass a 3D model of a patient's hip joint, complete with bone density information and optimal implant positioning, used in robotic-assisted hip replacement surgery. Additionally, this term could include an accurate digital replica of a patient's facial bones and soft tissues used for planning complex craniofacial reconstructions.

Furthermore, in the context of neurosurgery, the "three-dimensional digital model of the anatomical structure of interest" might refer to a detailed brain model showing tumor location, critical neural pathways, and blood vessels for planning and executing accurate tumor resections.

In practice, the rigid point-to-point registration process involves the probe tip touching predefined registration points on the patient's anatomy, which are then matched to predetermined points in the digital representation.

The "rigid" aspect of this registration implies that the spatial relationships between the points remain fixed and do not allow for deformation or scaling.

This rigid registration is typically implemented using robust mathematical techniques for finding the optimal rigid transformation between two sets of corresponding points. These techniques provide an efficient and accurate solution for aligning the patient's physical anatomy with the digital model.

This registration method ensures that the system 100 can accurately translate between the physical space of the patient and the virtual space of the digital model, which is essential for accurate guidance of the position-accurate robotic arm 111 and medical tools 112 during medical procedures.

The rigid registration process can be summarized in the following steps:

- collection of corresponding point pairs from the physical anatomy and the digital model,
- calculation of the centroids of both point sets,
- computation of optimal transformation matrices,
- determination of the optimal rotation and translation parameters.

This mathematical approach ensures a highly accurate and computationally efficient registration process.

Finally, the registration process enables the system 100 to establish an accurate spatial relationship between the physical anatomical structure and its digital representation. By utilizing the probe tip and predefined registration points, the system 100 ensures accurate alignment between the real-world patient anatomy and the three-dimensional digital model.

The implementation of advanced mathematical methods in the rigid registration process confers significant benefits to the system 100. These approaches minimize the distance between corresponding points, thereby ensuring optimal alignment between the physical and digital representations. The computational efficiency facilitates real-time registration updates during procedures when necessary, enhancing the system 100's responsiveness to dynamic surgical environments. Furthermore, the robustness to input data noise substantially improves the reliability of the registration process in clinical settings, where various factors may introduce minor discrepancies in measurements.

The implementation of the rigid registration method in the system 100 has significant implications for the overall performance and reliability of the medical procedures. This mathematical approach not only ensures accurate alignment between the physical anatomy and the digital model but also contributes to the system 100's ability to adapt to various clinical scenarios.

In the context of automated medical procedures, the registration method's ability to handle noisy data is particularly valuable. Medical environments can introduce various sources of error, such as minor patient movements or small inaccuracies in probe positioning. The algorithm's robustness helps mitigate these issues, maintaining registration accuracy even under less-than-ideal conditions.

Furthermore, the computational efficiency of the registration method allows for potential real-time updates to the registration during the procedure. This capability could be essential in scenarios where the anatomical structure of interest may shift slightly during the intervention, such as in soft tissue surgeries or procedures involving patient repositioning.

The integration of the registration method with other system components, including the tracking subsystem and the processor-based calibration module, establishes a comprehensive spatial awareness framework.

This integrated approach enables the system to perform continuous verification and updates of spatial relationships among all components.

Consequently, the system can adapt to subtle changes in the surgical environment without compromising its accuracy.

Moreover, this integration allows the system to provide real-time feedback to the position-accurate robotic arm 111, facilitating dynamic adjustments to the toolpath as required during the medical procedure.

These capabilities collectively enhance the system 100's ability to maintain accurate spatial relationships and execute accurate interventions throughout the duration of complex medical procedures.

▶**Enhanced Spatial Alignment System Using Point Cloud Registration for Comprehensive Anatomical Surface Mapping in Automated Medical procedures**

In an embodiment of the subject application, as illustrated in figure 7, the processor-based registration module 130 is further designed to perform an additional function in response acquiring surface data of predefined surfaces on the anatomical structure of interest with the probe tip, where the acquired surface data comprises information from at least two zones of one or more predefined surfaces on the anatomical structure of interest.

Specifically, the processor-based registration module 130 performs a point cloud registration between the acquired surface data and the three-dimensional digital model 20 of the anatomical structure of interest.

The term "point cloud registration" refers to an advanced computational process used by the automated system 100 to align a set of three-dimensional points, known as a point cloud, acquired from the surface of the patient's anatomical structure of interest with a corresponding set of points in the three-dimensional digital model. This process involves the probe tip collecting numerous data points from predefined surfaces on the patient's anatomy, which are then matched to the digital model 20 of the anatomical structure of interest using sophisticated algorithms.

The alignment is typically achieved using known iterative point matching methods, which progressively refine the transformation between the two point sets to minimize the distance between them. The point cloud registration provides a more comprehensive spatial mapping compared to the rigid point-to-point registration, as it captures the contours and topography of the anatomical surfaces rather than just individual points.

For example, the term "point cloud registration" may refer to the process of scanning or "painting" the surface of a patient's knee joint with the probe to create a dense set of data points, which are then aligned with the knee model derived from pre-operative imaging for accurate robotic-assisted knee replacement surgery. It may also encompass the act of capturing the contours of a patient's spine by running the probe along the vertebrae and matching this data to a spinal column model for accurate guidance in minimally invasive spinal medical procedures.

Additionally, this term could include the registration of a patient's facial features by acquiring surface data points and aligning them with a 3D facial model for planning and executing complex craniofacial reconstructions.

Furthermore, in the context of cardiac medical procedures, "point cloud registration" might involve mapping the internal surface of a heart chamber by collecting numerous data points with a specialized probe and aligning this information with a pre-operative 3D heart model for accurate catheter navigation during electrophysiology studies or ablation medical procedures.

This point cloud registration process enhances the system 100's ability to account for subtle anatomical variations and potential tissue deformations, thereby improving the overall accuracy and reliability of the automated medical procedures.

Finally, the point cloud registration process enables the system 100 to establish a more comprehensive spatial relationship between the physical anatomical structure and its digital representation. By utilizing the probe tip to acquire surface data from predefined surfaces, the system 100 enhances the accuracy of the alignment between the real-world patient anatomy and the three-dimensional digital model.

►**Specialized Automated System for High-Precision Dental Medical procedures Utilizing Advanced Robotic and Tracking Technologies**

In an embodiment of the subject application, the system 100 is applicable to specific medical procedures. In particular, the medical procedures performed by the system 100 are dental medical procedures.

The term "dental medical procedures" refers to a specific subset of medical interventions that focus on the diagnosis, treatment, and prevention of conditions affecting the oral cavity, particularly the teeth and gums.

In the context of the system 100, dental medical procedures encompass a range of highly accurate and specialized interventions that are performed using the system 100's advanced capabilities, including the position-accurate robotic arm 111, tracking subsystem 120, and sophisticated registration processes. These medical procedures leverage the system 100's ability to navigate complex oral anatomies with high accuracy and repeatability, while maintaining a level of autonomy that enhances the precision and efficiency of dental treatments.

For example, the term "dental medical procedures" may refer to the accurate preparation of teeth for crowns or veneers, where the position-accurate robotic arm 111 can follow a pre-planned toolpath to remove exactly the right amount of tooth structure with minimal margin for error. It may also encompass the placement of dental implants, where the system 100 can use its tracking and registration capabilities to navigate the drill to the exact position and depth required for optimal implant placement.

Additionally, this term could include complex orthodontic medical procedures, such as the accurate placement of brackets or the creation of custom-fit aligners based on highly accurate 3D scans of the patient's dentition.

Furthermore, in the context of this system 100, "dental medical procedures" might involve minimally invasive periodontal treatments, where the position-accurate robotic arm 111 can perform tasks like targeted plaque and calculus removal with a level of precision that surpasses human capabilities. The system 100's ability to perform point cloud registration could be particularly valuable in medical procedures requiring extensive surface mapping, such as full-mouth rehabilitations or complex cosmetic dentistry cases, ensuring that the final result closely matches the pre-planned digital design.

► **Method for Operating an Automated Medical procedure System with Integrated Calibration, Registration, and Precision Control**

As shown in figure 8 the subject application also relates to a computer-implemented method 200 for operating the system 100 as described above.

The computer-implemented method 200 comprises several steps involving the calibrated robotic subsystem 110, the tracking subsystem 120, targets, and various processes for registration, calibration, and control.

The computer-implemented method 200 begins by providing 210 the calibrated robotic subsystem 110 as already described above.

Next, the computer-implemented method 200 involves providing 220 the tracking subsystem 120 as already described above.

The computer-implemented method 200 then requires positioning and fixing 230 the tracking device at a distance of at least a predetermined value from the patient. Importantly, this predetermined value is selected to allow both tracking and sufficient workspace for the position-accurate robotic arm

The computer-implemented method 200 continues by configuring 240 at least two targets. These targets are designed to be tracked without physical connection to the calibrated robotic subsystem 110. Each target has a factory-defined geometric configuration created by an arrangement of multiple tracking markers 10.

Among these targets, at least one robot target 122, with a first factory-defined geometric configuration, is attached to the position-accurate robotic arm 111. Additionally, at least one patient target 123, with a second factory-defined geometric configuration different from the first, is attached to or in proximity to a detachable element 150 fixedly secured to an anatomical structure of interest.

Following this, the computer-implemented method 200 involves building 250 a three-dimensional digital model of the workspace associated with the anatomical structure of interest. This model is built from images acquired by the tracking device 121.

The computer-implemented method 200 then requires performing 260 a registration process that systematically aligns coordinate systems and establishes transformations between the position-accurate robotic arm 111, the tracking device 121, and the anatomical structure of interest.

Subsequently, the computer-implemented method 200 involves storing 270 an initial factory calibration. This calibration establishes a spatial relationship between the calibrated robotic subsystem 110 and the tracking subsystem 120.

A key step in the computer-implemented method 200 is performing 280 systematic spatial recalibration of the calibrated robotic subsystem 110 and the tracking subsystem 120. This recalibration is performed as a preparatory step prior to initiating each medical procedure, as well as upon any tool 112 exchange.

The recalibration process includes comparing the current physical geometry of each target to its factory-defined geometric configuration to establish a new spatial relationship. Based on this comparison, new recalibration results are generated. These results are then used to implement the new spatial relationship between the calibrated robotic subsystem 110 and the tracking subsystem 120.

As a safety measure, the computer-implemented method 200 prevents 290 the start of any medical procedure until both the immediately preceding registration process and recalibration process are successfully completed.

Following recalibration, the tracking device 121 is reconfigured 291 to recognize and track the targets based on their current physical geometry, as determined by the new recalibration results.

The computer-implemented method 200 then proceeds to localize 292 each target within the three-dimensional digital model of the workspace.

Finally, the computer-implemented method 200 involves controlling 293 the position-accurate robotic arm 111. This control allows the arm to automatedly guide at least one mounted interchangeable medical tool 112 along a predefined toolpath linked to the patient's anatomical structure for executing medical procedures. The guidance utilizes the tool's defined center point, TCP, as a reference point, based on the tracked positions and the localized targets within the three-dimensional digital model.

▶**Method for Operating an Automated Medical procedure System with Integrated Calibration, Registration, and Precision Control**

In an embodiment of the subject application, the computer-implemented method 200 further includes an additional step to enhance patient stability during medical procedures.

Specifically, this step involves immobilizing the anatomical structure of interest using a rigid frame 160 as already described above.

▶**Probe-Based Rigid Point-to-Point Registration Method for Accurate Anatomical-Digital Alignment in Automated Medical procedures**

In an embodiment of the subject application, the computer-implemented method 200 further includes a specific process for performing registration using a probe with a defined tip. This registration process comprises several steps to ensure accurate alignment between the physical anatomical structure and its digital representation.

Initially, the computer-implemented method 200 involves touching 261 predefined registration points on the anatomical structure of interest with the probe tip. Following this action, the computer-implemented method 200 responds to the probe tip touching the predefined registration points.

Specifically, it performs 262 a rigid point-to-point registration between the acquired registration points and corresponding predefined points on a three-dimensional digital model 20 of the anatomical structure of interest.

By implementing this registration process, the computer-implemented method 200 establishes an accurate spatial relationship between the physical anatomical structure and its digital model.

▶**Enhanced Registration Method Using Surface Data Acquisition and Point Cloud Registration for Refined Spatial Alignment in Automated Medical procedures**

In an embodiment of the subject application, the computer-implemented method 200 further includes additional steps in the registration process to enhance its accuracy and comprehensiveness. Specifically, these steps involve acquiring surface data and performing point cloud registration.

Initially, the computer-implemented method 200 requires acquiring 263 surface data of predefined surfaces on the anatomical structure of interest with the probe tip. Following this data acquisition, the computer-implemented method 200 responds by performing 264 a point cloud registration. This registration occurs between the acquired surface data and the three-dimensional digital model 20 of the anatomical structure of interest.

By incorporating this point cloud registration, the computer-implemented method 200 further refines the spatial relationship between the physical anatomical structure and its digital representation.

►**Conclusion**

The description of the subject application has been presented for purposes of illustration and description but is not intended to be exhaustive or limited to the application in the form disclosed. The embodiments were chosen and described to better explain the principles of the application and the practical application, and to enable the skilled person to understand the application for various embodiments with various modifications as are suited to the particular use contemplated.

In particular, when an expression uses the term "at least one," it means that the element or characteristic in question may be present in a single occurrence or in multiple occurrences, thus including one, two, three, or more elements or characteristics, without a specified upper limit.

On the other hand, when an element is "designed" to fulfill a particular function, it means that this element is created specifically for the purpose of fulfilling this particular function.

However, depending on the needs and available resources, it may be considered to use an existing element, which will be modified or adapted to fulfill this particular function, without requiring substantial modifications to the subject application.

Regarding the expression "all or part," it indicates flexibility in the selection or use of the mentioned elements or data. This expression means that the described action or characteristic can apply to the complete set of elements or data in question, or only to a selected portion thereof. The use of "all or part" thus encompasses a wide range of possibilities, from full use to partial use, without specifying a precise lower or upper limit as to the quantity or proportion concerned.

It should be noted that the examples provided throughout this description are presented for illustrative purposes and are not limiting. These examples aim to facilitate the understanding of the subject application by the person skilled in the art, by providing concrete illustrations of possible implementation.

However, the subject application is not limited to these specific examples. The person skilled in the art will understand that these examples can be generalized, adapted, or modified according to specific needs, technological advances, or particular constraints, without departing from the spirit of the subject application. Thus, whenever an example is given, it should be interpreted as encompassing not only the specific example mentioned, but also all technically equivalent variants and alternatives that fulfill the same function or achieve the same objective in the context of the subject application.

The subject application may be subject to numerous variants and applications other than those described above. In particular, unless otherwise specified, the various structural and functional characteristics of each particular implementation described above should not be considered as combined and/or closely and/or inextricably linked to each other, but, on the contrary, as simple juxtapositions. Moreover, the structural and/or functional characteristics of the different embodiments described above may be subject in whole or in part to any different juxtaposition or any different combination.

**Claims**

1. A system (100) for performing automated medical procedures, the system (100) comprising,

   - a calibrated robotic subsystem (110) comprising,

     -- a position-accurate robotic arm (111), designed to operate with interchangeable medical tools (112), each tool (112) having a defined center point, TCP, the position-accurate robotic arm (111) being capable of automatedly guiding at least one mounted tool (112) along a predefined toolpath linked to an anatomical structure of interest of a patient for executing medical procedures, utilizing the tool's TCP as a reference point,

   - a tracking subsystem (120) comprising,

     -- a tracking device (121) designed to

       --- track multiple targets, and
       --- be positioned and fixed, at a distance of at least a predetermined value from a patient, wherein the predetermined value is selected to allow both tracking and sufficient workspace for the position-accurate robotic arm (111),
     -- at least two targets designed to be tracked without physical connection to the calibrated robotic subsystem (110), each having a factory-defined geometric configuration created by an arrangement of a multiple tracking markers (10), allowing the tracking device (121) to locate each target within a three-dimensional digital model of the workspace associated with the anatomical structure of interest, built from images acquired by the tracking device (121), including,

--- at least one robot target (122), with a first factory-defined geometric configuration, designed to be attached to the position-accurate robotic arm (111), and

--- at least one patient target (123), with a second factory-defined geometric configuration different from the first factory-defined geometric configuration, designed to be attached to or in proximity to a detachable element (150) fixedly secured to anatomical structure of interest,

- a processor-based registration module (130) designed to perform a registration process to systematically align coordinate systems and establish transformations between the position-accurate robotic arm (111), the tracking device (121), and the anatomical structure of interest,

- a processor-based calibration module (140) including at least a memory, the processor-based calibration module (140) being designed to perform a recalibration process, including,

-- store an initial factory calibration establishing a spatial relationship between the calibrated robotic subsystem (110) and the tracking subsystem (120),

-- perform systematic spatial recalibration of the calibrated robotic subsystem (110) and the tracking subsystem (120) as a preparatory step prior to initiating each medical procedure, as well as upon any tool (112) exchange, wherein the recalibration process,

--- compares the current physical geometry of each target to its factory-defined geometric configuration to establish a new spatial relationship,

--- generates new recalibration results based on the comparison,

--- implements the new spatial relationship between the calibrated robotic subsystem (110) and the tracking subsystem (120) using the new recalibration results, and

--- proceed with the medical procedure using the newly spatially recalibrated system (100),

wherein,

-- the system (100) is designed to prevent the start of any medical procedure until both the immediately preceding registration process and recalibration process are successfully completed, and

-- the tracking device (121) is redesigned to recognize and track the targets based on their current physical geometry, as determined by the new recalibration results, ensuring accurate real-time tracking during the medical procedure.

2. The system (100) of claim 1 further comprising a rigid frame (160) designed to immobilize the anatomical structure of interest, so as to minimize unintended patient movement during medical procedures.

3. The system (100) of any one of claims 1 to 2 further comprising at least one probe, wherein the probe is either,

- one of the interchangeable medical tools (112) with a defined position at its tip, or
- a dedicated probe (170) associated with a third target, called pointer target (124), the pointer target (124) being comprised in the tracking subsystem (120), the dedicated probe (170) having a defined position at its tip.

4. The system (100) of claim 3, wherein the detachable element (150) comprises at least one calibrated recess (151) with predetermined characteristics, structured to engage all or part of the probe tip and allow rotational movement of the probe when the probe tip is engaged in the recess (151), such that the tracking device (121), in response to the rotation, can determine the position tip of the probe from the detected rotational characteristics in conjunction with either the robot target (122) or the pointer target (124), depending on whether the probe is one of the interchangeable medical tools (112) or a dedicated probe (170).

5. The system (100) of any one of claims 3 to 4, wherein the processor-based registration module (130) is further designed to, in response to touching predefined registration points on the anatomical structure of interest with the probe tip, perform a rigid point-to-point registration between the acquired registration points and corresponding predefined points on a three-dimensional digital model (20) of the anatomical structure of interest.

6. The system (100) of any one of claims 3 to 5, wherein the processor-based registration module (130) is further designed to in response to acquiring surface data of predefined surfaces on the anatomical structure of interest with the probe tip, perform a point cloud registration between the acquired surface data and the three-dimensional digital model (20) of the anatomical structure of interest.

7. The system (100) of any one of claims 1 to 6, wherein the medical procedures are dental medical procedures.

8. The system (100) of claim 7, wherein the tool (112) is selected among a dental drill for veneers or crowns, and an implant drill.

9. A computer-implemented method (200) for operating a system (100) for performing automated medical procedures, the computer-implemented method (200) comprising,

   - providing (210) a calibrated robotic subsystem (110) comprising a position-accurate robotic arm (111) designed to operate with interchangeable medical tools (112), each tool (112) having a defined center point, TCP,
   - providing (220) a tracking subsystem (120) comprising a tracking device (121),
   - positioning and fixing (230) the tracking device (121) at a distance of at least a predetermined value from the patient, wherein the predetermined value is selected to allow both tracking and sufficient workspace for the position-accurate robotic arm (111),
   - configuring (240) at least two targets to be tracked without physical connection to the calibrated robotic subsystem (110), each having a factory-defined geometric configuration created by an arrangement of multiple tracking markers (10), including,

      -- at least one robot target (122), with a first factory-defined geometric configuration, attached to the position-accurate robotic arm (111), and
      -- at least one patient target (123), with a second factory-defined geometric configuration different from the first factory-defined geometric configuration, attached to or in proximity to a detachable element (150) fixedly secured to an anatomical structure of interest,

   - building (250) a three-dimensional digital model of the workspace associated with the anatomical structure of interest from images acquired by the tracking device (121),
   - performing (260) a registration process to systematically align coordinate systems and establish transformations between the position-accurate robotic arm (111), the tracking device (121), and the anatomical structure of interest,
   - storing (270) an initial factory calibration establishing a spatial relationship between the calibrated robotic subsystem (110) and the tracking subsystem (120),
   - performing (280) systematic spatial recalibration of the calibrated robotic subsystem (110) and the tracking subsystem (120) as a preparatory step prior to initiating each medical procedure, as well as upon any tool (112) exchange, including,

      -- comparing the current physical geometry of each target to its factory-defined geometric configuration to establish a new spatial relationship,
      -- generating new recalibration results based on the comparison,
      -- implementing the new spatial relationship between the calibrated robotic subsystem (110) and the tracking subsystem (120) using the new recalibration results,

   - preventing (290) the start of any medical procedure until both the immediately preceding registration process and recalibration process are successfully completed,
   - reconfiguring (291) the tracking device (121) to recognize and track the targets based on their current physical geometry, as determined by the new recalibration results,
   - localizing (292) each target within the three-dimensional digital model of the workspace,
   - controlling (293) the position-accurate robotic arm (111) to automatedly guide at least one mounted interchangeable medical tool (112) along a predefined toolpath linked to the patient's anatomical structure for executing medical procedures, utilizing the tool's defined center point, TCP, as a reference point, based on the tracked positions and the localized targets within the three-dimensional digital model.

10. The computer-implemented method (200) of claim 9, further comprising immobilizing (294) the anatomical structure of interest using a rigid frame (160) to minimize unintended patient movement during medical procedures.

11. The computer-implemented method (200) of any one of claims 9 to 10, wherein performing (260) the registration process comprises using a probe with a defined tip, the computer-implemented method (200) further comprising,

      - touching (261) predefined registration points on the anatomical structure of interest with the probe tip, and
      - in response to the probe tip touching the predefined registration points, performing (262) a rigid point-to-point registration between the acquired registration points and corresponding predefined points on a three-dimensional digital model (20) of the anatomical structure of interest.

12. The computer-implemented method (200) of claim 9 to 11, wherein performing (260) the registration process further comprises,

- acquiring (263) surface data of predefined surfaces on the anatomical structure of interest with the probe tip, and
- in response to acquiring the surface data, performing (264) a point cloud registration between the acquired surface data and the three-dimensional digital model (20) of the anatomical structure of interest.

[Fig. 1]

Fig. 1

[Fig. 2]

Fig. 2

[Fig. 3]

Fig. 3

[Fig. 4]

Fig. 4

[Fig. 5]

121

170

150

151

Fig. 5

[Fig. 6]

112

20

1

Fig. 6

[Fig. 7]

Fig. 7

[Fig. 8]

Fig. 8

Europäisches
Patentamt

European
Patent Office

Office européen
des brevets

# EUROPEAN SEARCH REPORT

**Application Number**

EP 24 22 2649

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2024/008933 A1 (STAWIASKI JEAN [DE] ET AL) 11 January 2024 (2024-01-11) * especially: para. 47, 68, 71, 72, 79, 81, 93; paragraph [0010] - paragraph [0096]; figures 1-9B * | 1-12 | INV. A61B34/20 A61B34/30 A61B90/14 A61C1/00 ADD. |
| A | US 2022/125520 A1 (CRAWFORD NEIL R [US] ET AL) 28 April 2022 (2022-04-28) * paragraph [0059] - paragraph [0187]; figures 1-40 * | 1-12 | A61B17/00 A61B90/00 |
| A | US 2023/013550 A1 (BRIK ROBERT [US] ET AL) 19 January 2023 (2023-01-19) * paragraph [0005] - paragraph [0062]; figures 1-9 * | 1-12 | |

**TECHNICAL FIELDS SEARCHED (IPC)**

A61B
A61C

The present search report has been drawn up for all claims

| Place of search | Date of completion of the search | Examiner |
|---|---|---|
| Munich | 3 June 2025 | Kamp, Martin |

EPO FORM 1503 03.82 (P04C01)

## ANNEX TO THE EUROPEAN SEARCH REPORT
## ON EUROPEAN PATENT APPLICATION NO.

EP 24 22 2649

This annex lists the patent family members relating to the patent documents cited in the above-mentioned European search report.
The members are as contained in the European Patent Office EDP file on
The European Patent Office is in no way liable for these particulars which are merely given for the purpose of information.

03-06-2025

| Patent document cited in search report | | Publication date | Patent family member(s) | | Publication date |
|---|---|---|---|---|---|
| US 2024008933 | A1 | 11-01-2024 | DE 102019004235 | A1 | 16-01-2020 |
| | | | US 2020015909 | A1 | 16-01-2020 |
| | | | US 2022175467 | A1 | 09-06-2022 |
| | | | US 2024008933 | A1 | 11-01-2024 |
| US 2022125520 | A1 | 28-04-2022 | CN 114469347 | A | 13-05-2022 |
| | | | EP 3991683 | A1 | 04-05-2022 |
| | | | JP 7323590 | B2 | 08-08-2023 |
| | | | JP 2022070834 | A | 13-05-2022 |
| | | | US 2022125520 | A1 | 28-04-2022 |
| US 2023013550 | A1 | 19-01-2023 | NONE | | |

EPO FORM P0459

For more details about this annex : see Official Journal of the European Patent Office, No. 12/82